Europäisches Patentamt

European Patent Office

Office européen des brevets

(11) Publication number: **0 222 360**
A2

# (12) EUROPEAN PATENT APPLICATION

(21) Application number: 86115574.5

(22) Date of filing: 10.11.86

(51) Int. Cl.⁴: **A 61 K 47/00**
C 12 N 5/00, C 12 P 21/00

(30) Priority: 12.11.85 US 797431

(43) Date of publication of application:
20.05.87 Bulletin 87/21

(84) Designated Contracting States:
AT BE CH DE ES FR GB GR IT LI LU NL SE

(71) Applicant: Biotherapeutics Inc.
Riverside Drive
Franklin Tennessee 37064-5399(US)

(72) Inventor: Oldham, Robert K.
Route 2 Lewisburg Pike
Franklin Tennessee 37064(US)

(72) Inventor: West, William H.
4354 Walnut Grove Road
Memphis Tennessee 38117(US)

(74) Representative: Patentanwälte Grünecker, Kinkeldey,
Stockmair & Partner
Maximilianstrasse 58
D-8000 München 22(DE)

(54) A method of producing a patient-specific cytotoxic reagent and composition.

(57) A biotherapeutic process for preparing a patient-specific cytotoxic composition against a malignant tumor. Immune B-cell lymphocytes are obtained from in vitro and/or in vivo immunization with tumor cells. The immune B-cells are fused with myeloma cells or B-lymphoblastoid cells to form hybridomas which are then tested for production of monoclonal antibodies against the tumor cells. A plurality of selected hybridomas, each generating tumor-selective antibodies, are isolated and cloned and are used to produce monoclonal antibodies in large amounts. These antibodies are combined in a monoclonal antibody "cocktail" that will react with substantially all the tumor cells. The monoclonal antibody cocktail is linked with a drug, toxin, isotope or biological substance to form an immunoconjugate preparation which can be used, after appropriate preclinical safety and activity testing, for treating the patient with a custom-tailored therapeutic agent.

EP 0 222 360 A2

./...

BIOTHERAPEUTIC CANCER TREATMENT PROCESS

obtain tumor cell

→ preserve tumor cell

grow in vivo — culture in vitro — make antigen preparation

tumor cell preparation 100

spontaneous patient B-cell production — culture in vitro — immunise animal

extract patient B-cells — extract human B-cells — extract animal B-cells

fuse with B-lymphoblastoid cells or myeloma cells to form hybridomas

fuse with myeloma cells to form hybridomas

hybridoma generation 120

test hybridomas for antibody production

test antibodies against tumor cells

separate and clone effective hybridomas

generate antibodies from each hybridoma (in vivo or in vitro) — mix hybridomas

mix antibodies — generate antibodies from hybridomas (in vivo or in vitro)

monoclonal antibody cocktail

purify monoclonal antibody cocktail

antibody cocktail generation 130

link with drug — link with isotope — link with toxin — link with biologic

conjugation 140

immunoconjugate

purify immunoconjugate

test localization and effectiveness in tumor-bearing animal

test safety in laboratory animal

test localization and effectiveness in patient

testing 150

administer to patients

therapy 160

FIGURE 1

**0222360**

Biotherapeutics Inc.
Riverside Drive
Franklin
Tennessee 37064-5399
U. S. A.

EP 3238-60

A METHOD OF PRODUCING A PATIENT-SPECIFIC CYTOTOXIC REAGENT AND COMPOSITION

BACKGROUND AND PRIOR ART

The present invention relates to the field of monoclonal antibodies. In particular, the invention concerns the production of a combination of monoclonal antibodies, each being selective to a given tumor cell type for which hybridoma cells are developed. The antibody combination is conjugated with reagents which are toxic to or otherwise reactive with the tumor and may produce therapeutic benefits.

The three traditional modalities of cancer treatment, surgery, radiotherapy and chemotherapy have proven roles in cancer treatment. New surgical techniques have been developed both for resectable tumors and for use in combined-modality treatment to allow for more effective radiotherapy and chemotherapy. New methods of radiotherapy, new machinery for better delivery of radiotherapeutic agents, and drugs which can protect or enhance radiation effects have been developed. Unfortunately, even when applied in the most optimal fashion, over half of the patients with cancer still remain incurable, primarily because of resistant, metastatic disease, requiring systemic treatment. Oldham, Cancer Treat. Rep., 68:221-232 (1984).

Although chemotherapy can effectively be applied systemically, the problems of normal tissue toxicity, drug delivery and tumor cell resistance limit the effectiveness of this approach. Current modes of systemic treatment are very nonselective and have considerable toxicity to normal tissues as well as cancer tissues. Depending on how one selects the normal tissue control, toxicity may actually be greater to normal tissue than to the malignant tissue. Even in the best of circumstances considerable toxicity to normal tissue accompanies effective cancer treatment with the currently available drugs. Yet, it is important to deliver drugs to human tumor cells in adequate concentrations to overcome drug-resistant characteristics of the cells. Hanna et al, *J. Biol. Resp. Modif.*, 4:295-309 (1983). Therefore, it is apparent that there is a need for a more selective approach in systemic cancer treatment in order to deliver drugs and other toxic agents specifically to cancer cells in the doses needed.

Antibodies are proteins made by B-lymphocytes or B-cells that react with antigen determinants and form the basis of the humoral immune system. Antibodies have long been known as biological agents with the potential for specificity in recognizing that tumor tissue is different from normal tissue. A major limitation for the use of antibodies, however, was the inability to make repetitively high-titer, specific antisera on a large scale, in a cost effective manner and to define these preparations on a molecular basis.

A major advance in the prospects for using antibodies for cancer treatment was made by the development of monoclonal antibodies having a great degree of specificity for tumor cells. Oldham, *J. Clin. Onc.*, 1:582-590 (1983). The technique of hybridization of an immortal myeloma cell line with an antibody-producing B-cell, was developed by Kohler and Milstein. Kohler et al, *Nature*, 256:495-497 (1975). With the advent of this hybridoma-monoclonal antibody technology, immunoglobulin reagents can now be produced with the same level of molecular purity as that for cloned gene products and for drugs (synthetic chemicals). Substantial quantities of antibody preparations can now be made which are specific to one or more antigens and/or antigenic epitopes on the tumor cell membrane. Koprowski et al, U.S. Patent No. 4,172,124. Thus, a strong scientific base has been established for the use of biologicals and biological response modifiers, particularly monoclonal antibodies, as a fourth modality for treating cancer. Oldham, Cancer Treat. Rep., 68:221-232 (1984).

Most of the early monoclonal antibody research has focused on the use of unconjugated antibody to determine toxicity, tolerance and localization of antibody in solid tumors and the distribution of antibody in normal and neoplastic tissues. Oldham and co-workers were among the first to publish unequivocal evidence that antibody does find tumor locations and can localize on individual tumor cells after intravenous injection into patients with solid tumors. Oldham, J. Clin. Onc., 1:582-590 (1983); Oldham et al, J. Clin. Onc., 2:1235-1242 (1984); Schroff et al, J. Nat. Cancer Inst., 74:299-306 (1985). Biodistribution studies utilizing antibody conjugated to tracer quantities of isotope have also demonstrated that a considerable amount of antibody will traffic to the liver and spleen and other organs of the reticuloendothelial system. Goldenberg et al, J. Biol. Resp. Modif., 1:121-136 (1984); Bernhard et al, Cancer Res., 43:4429-4433 (1983).

Most of the monoclonal antibodies developed against human tumor cells have been made in mice by immunizing a mouse with human tumor cells, tissue culture lines developed from tumor cells and/or extracts thereof. These murine (mouse) monoclonal antibodies are reasonably well tolerated by patients when given in several injections over a few weeks, and there is selective retention of the antibody or antibody isotope conjugate in the area of the tumor. Selected antibodies will localize rather strikingly on tumor cells and will be retained there much longer than in normal tissues to which they may also have some affinity. These findings indicate that antibody preparations may be useful in the selective targeting of toxins, drugs, radioactive isotopes and biologicals to solid tumors.

A major difficulty with using antibody alone appears to be that of antigenic modulation, resulting in internalization or shedding of antigen and of the cell surface-bound

0222360

monoclonal antibody. Thus far, more antigenic modulation has been observed with the antigens on hematopoietic cells than with those on solid tumors. While modulation may be a limiting problem for antibody alone, it may be a positive factor for immunoconjugate therapy, where the toxicity of immunoconjugates may occur prior to or during modulation of the tumor cell. In fact, modulation may be a major mechanism by which immunoconjugated molecules, gain entrance into the cell and cause their toxic effects. Oldham, Cancer Treat. Rep., 68:221-232 (1984); Schroff et al, J. Nat. Cancer Inst., 74:299-306 (1985).

Early studies with the use of immunoconjugates of antibodies and isotopes in human solid tumors have shown encouraging localization and even some evidence of therapeutic effects. See Oldham, J. Clin. Onc., 2:1235 (1984). These data along with the historical data on the use of heteroantisera, drug and isotope conjugates indicate that monoclonal antibody immunoconjugates are likely to be very useful in improving the selectivity of cancer treatment. Oldham, Immun. to Cancer, 575-586, Academic Press (1985).

In spite of these encouraging data for the use of antibody as a targeting agent, the heterogeneity of cancer presents a serious problem. As with normal cells, cancer cells include a large variety of antigens, some of which are unique or represent unique arrays (quantitatively or qualitatively) for the tumor cells. Since any single tumor antigen may or may not be present on all tumor cells, it is unlikely that a singular and totally specific antibody can be identified that will target all of the tumor cells. Also, quantitative differences in antigens and antigenic arrays appear to exist on cancer cells.

Thus, the use of a preparation from a single antibody or a combination of one or two antibodies that cover only a portion of the tumor cells will not eliminate the complete replicating cell population from the tumor cell population present in the patient. The residual viable tumor cells are likely to grow and may be nonreactive with or resistant to those antibodies. Without antibody combinations which react with substantially all of these unique and selective tumor antigens, cancer treatment using monoclonal antibodies has not been effective.

## SUMMARY OF THE INVENTION

The present invention provides novel methods for producing cancer-selective cytotoxic reagents for the treatment of malignant tumors. In accordance with one aspect of the present invention, a tumor portion is obtained from a patient and is processed and/or expanded to form a tumor cell preparation. This preparation is used to immunize B-lymphocytes so as to produce antibodies which are selective for the tumor cells. The B-lymphocytes are isolated and fused with fusion partner cells to form hybridomas. Each hybridoma is tested to determine whether it produces antibodies which are selective for the tumor cells. The hybridomas which produce these selective antibodies are isolated and cloned so that a substantial amount of a plurality of antibody types can be produced which are selective for the tumor cells. These monoclonal antibodies are then combined to generate a monoclonal antibody "cocktail" that will react with substantially all of the tumor cells. The monoclonal antibody cocktail is linked with a substance which is toxic to or otherwise reactive with the tumor cells to provide an immunoconjugate reagent for treating the tumor.

With respect to more specific embodiments of the present invention, the tumor cell preparation is preferably generated by culturing the tumor cells in vitro or developing an antigen preparation from the membranes of the tumor cells. The tumor cells may also be grown in vivo, preferably as subcutaneous implants in immunodeficient (Nu/Nu) mice.

The immunized B-lymphocytes are preferably generated by injecting the tumor preparation into mice. The spleen cells of the animal are then removed and fused with myeloma cells to form hybridomas. Alternately, B-lymphocytes can be generated by culturing the tumor cell preparation with human or mouse lymphocytes in vitro or by extracting human B-lymphocytes which have been spontaneously produced or induced by immunization with tumor cells or tumor cell products (often with adjuvants) by or in the patient. Most preferably, hybridomas are formed by fusing extracted human B-cells with B-lymphoblastoid cells.

The hybridomas are tested for the production of antibodies which are then exposed to the tumor cells to determine which of the antibodies are selective to the tumor cells. The hybridomas producing these selective antibodies are isolated and cloned by known laboratory procedures. Then, antibodies are generated from the hybridomas, either in vitro or in vivo, to produce a monoclonal antibody cocktail that will react with substantially all of the tumor cells. This monoclonal antibody cocktail is purified and linked to a substance which is toxic to or otherwise reactive with the tumor cells to form an immunoconjugate preparation for use in treating the tumor. Preferably the substance is a drug, toxin, radioactive isotope or biologic. Radioactive isotopes are also useful in tracing activities (tumor diagnosis and staging). The immunoconjugate is then tested for localization and effectiveness, first in a tumor-bearing animal, preferably an animal bearing the patient's tumor, and then in the patient.

The foregoing invention provides a combination of a plurality of monoclonal antibodies, each being selective for the antigens of certain of the tumor cells of the patient. The combination of these plurality of monoclonal antibodies provides a cocktail which is specifically tailored to select for substantially all of the tumor cell types in the patient's tumor. Thus, the present invention provides a method for delivering toxic substances selectively to cancer cells so as to treat substantially all of the cancer cells while minimizing general toxicity to the patient's normal cells. By utilizing the tumor cells of the patient to generate tumor cell selective antibodies, delivery of toxic substances to substantially all of the tumor cells is insured. The use of hybridomas to produce the cancer-selective antibodies enables a ready and continual supply of relatively pure antibody which can be stored if necessary and used later as therapy requires. These cancer-selective antibodies can also be used more generally to type individual patient's tumors giving an alternative and additional method of antibody cocktail generation i.e., by typing tumors with existing antibodies "off the shelf". Thus, by using the patient's tumor as the start of the cancer treatment process, the present invention provides a custom-tailored monoclonal antibody cocktail which is selective for substantially all of the tumor cells of the patient and is toxic to the tumor cells to enable effective therapy while minimizing toxicity to the patient's normal cells.

## DETAILED DESCRIPTION OF
## PREFERRED EMBODIMENTS OF THE INVENTION

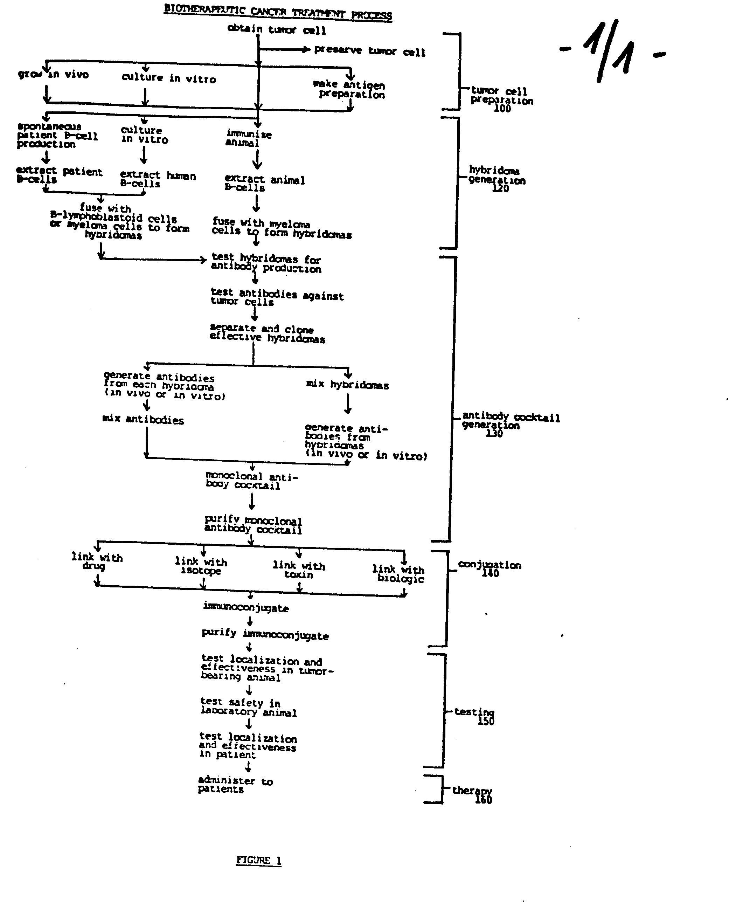

The following is a detailed description of preferred embodiments of the present invention. Figure 1 is a flow diagram showing the major steps of the preferred processes comprising this invention. The processes include the steps of generating a tumor cell preparation from the tumor of the patient, generating hybridomas from B-lymphocytes immunized by the tumor cells, producing a plurality of monoclonal antibodies from the hybridomas, combining the monoclonal antibodies in a cocktail form which will react with substantially all of the tumor cells and conjugating the monoclonal antibody cocktail with one or more substances which are toxic to or otherwise reactive with the tumor cells.

### Tumor Cell Preparation

With reference to Fig. 1, tumor cell preparation 100 is carried out by the steps shown therein. A tumor biopsy is effected for a patient suffering from malignant cancer. Preferably, a portion of the tumor sample is cryopreserved for later use, using the technique described in Oldham et al, Int. J. Cancer, 18:145-155 (1976) the teachings of which are hereby specifically incorporated by reference. A portion of the tumor cell is injected into nude mice for growth of the tumor cells in vivo, and another portion is cultured in vitro to enlarge the mass of cells available for later testing and immunizing. An antigen preparation is made by disaggregating the tumor cells and utilizing the external surface or membrane of the cancer cells, using the techniques disclosed in Morgan, Jr. et al, Hybridoma, 3:233 (1984), the teachings of which are hereby specifically incorporated by reference. The membrane of the cancer cell carries the antigens and is often more immunogenic than other portions of the cell. Also, the membrane is the preferred site of attachment for the below-described cancer-selective immunoconjugates.

Tumor cells may also be cultured in vitro in the presence of gamma interferon to induce novel antigen expression. In this process, human tumor cells are incubated in the presence of gamma interferon. These interferon stimulated tumor cells are then used for intraperitoneal or intravenous immunization of animals.

### Hybridoma Generation

Referring next to the steps involving hybridoma generation 120, a portion from the tumor biopsy or the antigen preparation is injected directly into experimental mice, preferably BALB/c type, to prepare immunized murine B-lymphocyte cells. After about ten days, the BALB/c mice are boosted with another injection of the tumor cells. Subsequently and some-times after further doses of a tumor preparation, the spleens of the BALB/c mice are removed and minced to make a single cell suspension. This suspension is washed and the red cells are removed to make a more purified lymphocyte preparation.

An alternative preferred method of immunization is to immunize B-lymphocytes in vitro, that is in culture medium with B-lymphocytes such as those found in spleen cells or in peripheral blood cells from patients with cancer. Methods for such in vitro immunization are well known, e.g., Foon et al, Monoclonal Antibody and Cancer, 143-155 (1983), the teachings of which are hereby specifically incorporated by reference.

Another preferred method of obtaining B-lymphocyte cells is through production by the patient. The patient has

0222360

already been immunized by his own cancer and in many cases spontaneously produces B-lymphocytes as a reaction to the tumor. In addition, the patient's immunological response can be stimulated by injecting specially prepared inactivated tumor cells intradermally. In either case, immunized B-lymphocytes are withdrawn from the patient's body, preferably from the spleen, peripheral blood cells or lymph nodes.

The hybridomas are preferably formed by fusing the extracted B-lymphocyte cells with a fusion cell partner. For murine B-cells the hybridoma is formed preferably by fusion with murine myeloma cells. For human B-cells, the hybridoma is formed preferably by fusion with human B-lymphoblastoid or myeloma cells. In either case, the fusion is performed in the presence of polyethylene glycol (PEG 1000-4000) using conventional processes such as those described in EMBO, Hybridoma Techniques (Cold Spring Harbor Laboratory, 1980), the teachings of which are hereby specifically incorporated by reference. Alternately, a newer technique called electrofusion can be used, as described in Kuta et al, ABL May/June 1985, 31-37.

The cells are then placed into a medium which enables the growth of only hybridoma cells made up of B-lymphocytes and myeloma cells or B-lymphoblastoid cells. Preferably, the cells are placed in a HAT medium which contains hypoxanthine, aminopterin and thymidine since only hybrids between B-lymphocyte cells and myeloma cells or B-lymphoblastoid cells survive in that medium. After about 7-10 days, the surviving hybridomas are removed and tested for specific antibody production.

Preferably, myeloma cells or B-lymphoblastoid cells are used as fusion partners with B-lymphocyte cells to form hybridomas because they secrete little if any immunoglobulin while living indefinitely. Thus, by being formed from myeloma cells or B-lymphoblastoid cells the hybridomas are given "eternal life" while secreting only antibodies specific to the immunized B-lymphocytes. It is understood that other fusion partner cells can be used which have similar characteristics.

The hybridomas are tested for antibody production by sampling the supernatant of the hybridomas using an assay that will measure immunoglobulin secretion. Preferably, this process is carried out by adding the supernatant to an antibody against immunoglobulin linked with an enzyme. Reaction against the immunoglobulin is measured by an absorbence detector using conventional techniques.

The antibodies produced by the hybridomas are tested for selectivity against the patient's tumor cells using several different procedures including plaque assays, agglutination, absorbtion, radio-immunoassays, enzyme immunoassays and enzyme-immunoglobulin coupling, e.g., Morgan et al, _Protides of the Biological Fluids_, 32:773-777 (1985), the teachings of which are hereby specifically incorporated by reference. In the assay process, the tumor cell or the antigen preparation is distributed in small aliquots in a microtiter plate made of plastic to which the antigen becomes absorbed. The test supernatant is added and incubated. If there is a reaction, the antibody will bind to one or more antigens on the tumor surface. The unbound antibody is washed away and the bound antibody is detected by conventional assay procedures. For example, the plastic may be incubated with radio-labeled or enzyme-labeled antibody directed against antibody produced by the fused cells. The plate is again washed and the microtiter wells are counted for radioactivity or incubated with enzyme substrate to determine reactivity of the antibody to the tumor preparation.

After the hybridomas which produce antibodies against the tumor cells have been isolated, the hybridomas must be isolated and individually cloned. It is important to clone separate positive cultures so that separate single cell cultures are grown which will each produce antibodies of one specific type. Cloning can be accomplished under limiting dilution conditions whenever the fraction of specific antibody secreting hybrids is very low and the cells to be cloned are not yet well adapted to tissue culture conditions. Though this procedure is time-consuming, it has often been the only way to establish a monoclonal specific antibody secreting line. A very dilute suspension of the cells is made in which the probability of having one cell per microtiter well is high. Several dilution tests may be required in order to achieve a sufficient number of microtiter wells having a single cell therein. Detection of the presence of particular types of antibody can be accomplished using the antibody-enzyme linked process described earlier.

Alternatively, isolation and cloning of individual hybridoma cells can be accomplished using a fluorescence-tagged antigen and laser counting techniques such as those used in cytofluorometry. Dangle et al, J. Immunological Methods, 52:1-14 (1982).

### Monoclonal Antibody Cocktail Generation

After the selected hybridomas have been cloned and grown in vitro, the antibody "cocktail" is generated using the process 130 shown in Fig. 1. The antibody "cocktail" generation process 130 includes screening to select hybridomas, and preparation to grow up and combine hybridomas and antibody. Antibodies from each of the different types of hybridomas are produced either in vivo or in vitro. Using the in vivo approach, the hybridoma cells are injected into the peritoneum cavity of BALB/c mice which have been pre-injected with a substance to stimulate the production of ascites fluid. Preferably a large number of mice are used in a given production to generate a substantial quantity of ascites fluid in a relatively short period of time. For example, injection of hybridoma solution into 200 mice will produce about one liter of ascites fluid in 7 to 10 days. After purification, the process yields up to 5 grams of antibody. Abrams et al, J. Immunol., 132:1611-1613 (1984).

The selected hybridomas may be mixed before injection in each mouse to yield an antibody cocktail from each mouse of all types of antibody which have been found to be selective for the tumor in question. Alternately, different

selected hybridomas may be injected separately into different mice so that the antibody types are produced separately. After the antibodies are withdrawn they are combined to form a cocktail of antibodies, all of which will select for and react with the tumor cells. One problem with the former approach is that by combining hybridomas prior to the antibody production, hybridoma types which have a higher rate of growth may produce more antibody than others, so that an uneven distribution of antibody may result. By producing each type of antibody separately, the proportion of each antibody in the resultant cocktail can be more carefully controlled and can be specifically formulated based on the quantitative relativity of the different tumor cell populations in the patient's cancer.

The production of a monoclonal antibody according to the present invention can also be carried out in vitro by placing hybridomas in tissue culture and harvesting the antibody from the supernatant of the culture. Typically, a culture medium of RPMI-1640 or other kind of formulation is used to make the cells grow and produce antibodies. Similarly, the antibody production can be carried out with all selected types of hybridomas combined or each type of hybridoma may be cultured separately with the resultant antibodies being combined thereafter.

In either event, the result of the foregoing antibody production process is a monoclonal antibody cocktail having a number of different types of antibodies therein. Each antibody type has been produced as a result of hybridomas which were generated specifically from one or more type of immunized B-lymphocytes taken directly as a result of immunization by the patient's tumor cells. As such, the antibodies are highly selective for certain determinants of the tumor cell antigen and are therefore custom-produced and/or custom selected for the patient's tumor. Moreover, the combination of each of the different antibodies produced enables a resultant antibody cocktail which is reactive with substantially all of the cells of the tumor of the patient being treated.

-13-    0222360

This monoclonal antibody cocktail is then purified through conventional processes. For example, after the ascites fluid is removed from the mice, it is centrifuged to remove any particulate matter or cells. A series of conventional biochemical steps are carried out involving precipitation of the antibody using sodium or ammonium sulfate combined with absorption of the antibody, using materials such as protein-A linked to sepharose. The protein-A binds certain antibodies which can then be recovered by changing the pH. Another method of purification involves using high performance liquid chromatography (HPLC), as described by Oldham et al, J. Clin. Onc., 2:1235-1242 (1984), a process which purifies monoclonal antibodies from the ascites fluid almost directly after ammonium sulfate separation.

The resultant monoclonal antibody cocktail is typically composed of from 3 to 10 different antibodies, depending on the heterogeneity of the tumor. It is possible, although not likely, that the number of types of antibodies present could exceed 10 in certain situations. The preparation is custom-tailored and as such, is different for each patient.

In another embodiment of the present invention, the monoclonal antibody cocktail is generated by selecting the desired hybridomas from a panel or bank of previously generated and preserved hybridomas by reacting antibodies with or typing each patient's tumor. The selection process is similar to that described above except that the hybridomas are already available without the necessary generation from a given patient's tumor cells. As hybridoma banks develop, they will be used more extensively for hybridoma sources in order to shorten substantially the amount of time involved in preparing antibodies for cancer treatment. In any event, the resultant monoclonal antibody cocktail is custom-tailored to be patient-specific to substantially all of the tumor cells of a given patient's cancer.

Immunoconjugate Generation

Referring again to Fig. 1, the conjugation process 140 is next carried out. The monoclonal antibody cocktail locates and targets substantially all of the tumor cells to deliver a substance which is toxic to the tumor and will cause it to regress. Preferably the monoclonal antibodies in the cocktail are linked to a drug, toxin, radioactive isotope or a biologic substance for this purpose. This linking process is carried out using conventional biochemical techniques, e.g., Hwang et al, Cancer Res., 44:4578-4586 (1984), the teachings of which are hereby specifically incorporated by reference. Any biologic, chemical or organic compound may be utilized which is reactive with and harmful to tumor cells but which may have toxicity to normal bodily cells. However, the need for concern about damage to normal cells is minimized because of the high selectivity of the monoclonal antibody cocktail of the present invention which enables the toxic substance to be preferentially localized to the tumor. Use of a radioisotope is apparent as a tracer for determining localization and effectiveness. However, isotopes can also be utilized as a substance for causing regression of the tumor in certain circumstances. Foon et al, J. Biol. Resp. Modif., 1:277-304 (1982).

The immunoconjugate is then purified through conventional biochemical processes similar to those described above for the monoclonal antibody cocktail.

Although the immunoconjugate is preferably made by linking as described above, it is also anticipated that recombinant monoclonal antibodies may be used which can bind drug or tracer substances biologically rather than through chemical coupling. See, e.g. Reading, U.S. Pat. No. 4,474,893.

### Testing

After the immunoconjugate solution has been prepared, it is tested for localization and effectiveness preferably according to steps 150 in Fig. 1. Oldham et al, J. Clin. Onc., 2:1235-1242 (1984). A portion of the patient's tumor tissue is transplanted into several BALB/c mice under renal capsule or transplanted as a xenograft subcutaneously in immunodeficient

(Nu/Nu) mice. The immunoconjugate preparation, preferably linked with a radioisotope, is injected into the mice and traced to determine whether a substantial portion of it localizes on the tumor. Testing is also carried out for effectiveness of the immunoconjugate solution, preferably linked with a toxin, drug, isotope or biologic, on slowing the rate of growth of the tumor or causing it to regress.

A safety test is also carried out by injecting a substantial overdose of the preparation in an experimental animal, preferably a BALB/c mouse. The animal does not have to have a tumor implant because the test is for toxicity and adverse effects on the varied systems in the animal, not on the tumor tissue.

After the immunoconjugate preparation has been determined to be safe and effective, it is administered in small doses to the patient to determine whether its localization characteristics carry over to the patient. If so, the preparation is administered in therapeutic doses and testing is carried out to determine the extent of regression of the tumor.

The present invention will be described in more detail with reference to the following examples. These examples are merely illustrative of the present invention and are not intended to be limiting.


EXAMPLE 1a

Tumor Cell Preparation (Solid Tumor):

A fresh clinical specimen of a histologically proven tumor tissue was disaggregated by mincing in RPMI 1640 medium supplemented with 5% penicillin - streptomycin - neomycin (Gibco, Grand Island, NY). The specimen was first trimmed of the vascularized connective and fibroadipose tissues. After cutting into small pieces of approximately $1mm^3$, the disaggregated cells were washed and put into culture in RPMI 1640 supplemented with 20% heat inactivated fetal bovine serum (Gibco). Some of the remaining chunks were also cultured.

Additional cells and chunks were cryopreserved. Some of the suspension was injected into athymic nude mice which were obtained from specific pathogen-free facilities (Animal Production Facility, NCI-FCRF, Frederick, MD). Selected nude mice were treated with estrogen pellets or antibody to reduce natural killer cell activity. Tumors were allowed to grow to $1-2$ $cm^3$ to generate a large tumor cell source for reimplantation, immunoperoxidase or a fluorescent histology and for making membrane preparations (Example IIa). Tumors were removed aseptically and frozen at $-70°C$ until further processing was required.

The tumor explant cultures were maintained in a humidified incubator at $37°C$ with a 95% air 5% $CO_2$ atmosphere and were observed daily. The cultures were generally mixtures of fast growing fibroblasts and slower growing tumor cells. The epithelioid tumor cell colonies were isolated with a 20 gauge, 1½-inch stainless hypodermic needle. After release into the medium, tumor cell aggregates were transferred to new flasks. This procedure was repeated until confluent monolayers of epithelial-like tumor cells were observed (1-2 months). For subsequent passage of the tumor cell lines, 0.25% trypsin-0.2% EDTA solution (Gibco) or a cell scraper were used.

<u>EXAMPLE 1b</u>

Tumor Cell Preparation (Ascites):

Two liters of ascites fluid were obtained from a patient with metastatic breast cancer. Twelve grams of red and white blood cells were obtained from this fluid by centrifugation at 1500 rpm for 30 minutes. Approximately $2 \times 10^8$ tumor cells/ml were recovered with a viability of 86%. Ten vials (1 $\times 10^8$ viable cells/vial) of this specimen were cryopreserved for later use. Two 75 $cm^2$ flasks were inoculated with cells and put into culture with RPMI 1640 medium supplemented with 5% penicillin-streptomycin-neomycin mixture (Gibco, Grand Island, NY), and 20% heat inactivated fetal bovine serum (FBS, Gibco). The cultures were maintained in a humidified incubator at $37°C$ with 95% air 5% $CO_2$ and were observed daily. Growth medium was replaced as needed. Within one week, a short term cell culture was established.

## EXAMPLE 1c(1)

Tumor Cell Antigen Preparation (lung cancer):

The antibody 81A6 generated against small cell carcinoma recognizes a neuroendocrine antigen widely expressed by small cell lung cancers. While the 81A6 antibody recognizes a shared antigen common to many tumors, this same antigen appears to express private epitopes that are unique to an individual patient's tumor. To prepare a custom tailored cocktail of antibodies against these epitopes, the patient's tumor tissue was lysed in n-octyl $\beta$-D-gluco pyranoside detergent (Sigma). Cellular debris was removed by centrifugation and this lysate was passed over an immunoaffinity column consisting of the 81A6 antibody coupled to cyanogen bromide-activated Sepharose™ 4B (Pharmacia, Piscataway, NJ). After thorough cleansing of the column, purified antigen was eluted from the column and absorbed onto lentil lectin-Sepharose™ 4B (lentil lectin was obtained from Sigma Chemical Co., St. Louis, MO) by gentle overnight rocking. Animals were then immunized with the Sepharose 4B lentil lectin-antigen complexes weekly for three weeks with a final intraperitoneal and intravenous boost at week six. Three days later the fusion procedure was completed. By screening against a membrane preparation of the patients' tumor in an enzyme-linked immunoassay, a panel of antibodies of various isotypes were developed for delivery of a custom-tailored cocktail preparation for optimal targeting of the patients' tumor cells.

## EXAMPLE 1c(2)

Tumor Cell Antigen Preparation (Interferon):

Human tumor cells growing at $5 \times 10^6$ per ml were incubated in the presence of gamma interferon at 100 units interferon per ml for 24 hours at 37 degrees under 5% $CO_2$. At the end of this culture period the tumor cells were washed twice in phosphate buttered saline (PBS) and resuspended at $5 \times 10^6$ per ml.

## EXAMPLE 1d

Tumor Cell Preparation (lymphoma):

For patients with B-cell lymphoma the target antigen is well defined and consists of the monoclonal immunoglobulin expressed in the cytoplasm and on the cell membrane of the malignant B-lymphocyte. A population of intact malignant B-lymphocytes can be used as the immunogen but this involves immunization of animals against a variety of irrelevant antigens. Accordingly, for certain patients it is preferable to induce immunoglobulin secretion by malignant cells in vitro and use purified monoclonal immunoglobulin as the immunogen.

To carry out such a process, the cell suspension from lymph nodes involved with B-cell malignancy or from the peripheral blood of patients with the leukemic phase of a B-cell malignancy were adjusted to a concentration of $2.5 \times 10^6$ cells per milliliter and mixed with an equal volume of 5% sheep red blood cells treated with 2-aminoethylisothiouronium hydrobromide (AET) which may be obtained from Sigma Chemical Co., St. Louis, Missouri. This mixture was centrifuged for ten minutes at 400g and maintained on ice for one hour. T-cells infiltrating the B-cell population formed rosettes with sheep red blood cells and were removed by centrifugation over a Ficol-Hypaque gradient at 400g for 30 minutes (a Ficol-Hypaque gradient may be obtained from Sigma Chemical Co., St. Louis, Missouri, under the tradename Histo-paque-1077).

After the patient's T-cells were removed, a population of normal T-cells separated from the peripheral blood mononuclear cells in the normal individuals and documented to contain less than 1% normal B-cells were added to the malignant B-cell population at a 1 to 1 T-cell to B-cell ratio. The normal T-cells were added because they have been previously demonstrated to provide help for immunoglobulin secretion by the neoplastic B-cells as described by Braziel, et al, Blood, 66:128-134 (1985). The cell population was then exposed to 12-O-tetradecanoylphorbol 13-acetate (TPA, phorbolester; which may be obtained from Sigma Chemical Co., St. Louis, MO) which is known to promote _in vitro_ differentiation and immunoglobulin production. The final concentration of TPA was $1.6 \times 10^{-8}$ mole per liter.

This mixture of malignant B-cells, normal T-cells and TPA was left in culture for 7 days. At the end of that 7 day period culture cells were centrifuged for 30 minutes into a pellet at 200g. The conditioned media containing secreted immunoglobulin was then passed over an immuno-affinity column consisting of sepharose beads coated with mouse monoclonal antihuman immunoglobulin antibody. The cell pellet was lysed in 0.5% NP-40 detergent which may be obtained from Sigma Chemical Co. and the cell lysate was also passed over the immuno-affinity column. After extensive washing, the sepharose gel beads coupled to mouse anti-human immunoglobulin which captures the patient's monoclonal immunoglobulin marker were removed from the column and were used for direct immunization of mice. Mouse monoclonal anti-human immunoglobulins were used to coat these beads and to capture the patient antibody because mouse immunoglobulin is relatively non-immunogenic compared with antibodies of other species.

## EXAMPLE 1e

Tumor Cell Antigen Preparation (Lymphoma - Rescue Fusion):

Fusion parent cells (SP2/0,HF2) obtained from the American Type Culture Collection, Rockville, MD (ATCC) were grown and collected from flasks and washed 3 times in RPMI-1640. Washed human tumor cells were collected from single cell suspension and both fusion parent cells and tumor cells were counted. Washed and counted cells were held on ice while counting was conducted. Appropriate dilutions were made and 3 $\times 10^7$ human lymphocytes were placed with $1 \times 10^7$ fusion partner cells in a 15ml round bottom tube.

After centrifuging for 5 minutes at 250 x g all media was carefully removed. 0.2ml of 35% PEG (polyethylene glycol 1450, Aldrich) was added per tube for 1 minute at room temperature and sufficient agitation was used to resuspend the pellet in PEG. The PEG was quickly diluted with 10ml RPMI-1640 and was let stand for 10 minutes at room temperature. The fusion tubes were centrifuged for 5 minutes at 250 x g and all dilute PEG was carefully removed. Each pellet was gently resuspended in 5 ml RPMI-1640 with 15% fetal bovine serum, the total volume was brought to 21 or 31ml in a 50ml conical, and was plated at 0.1ml/well in two or three 96-well tissue culture plates. The plates were incubated overnight at 37°C in a humidified chamber of 6% $CO_2$. After 18 to 24 and 0.1ml of 2 x HAT (hypoxanthine, aminopterin, thymidine) was added per well. Single colony wells were screened for secretion of idiotype after 2 to 3 weeks. Forty percent of the wells were reactive with the immunizing human antibody molecule. Of those positive colonies, 25% were reactive with the idiotype of the patient's antibody and were unreactive with other antibody preparations.

EXAMPLE 2a

Hybridoma Generation (Whole Cell Antigen):

Between 1-3 x $10^6$ viable tumor cells obtained in Example 1a above were injected intraperitoneally into each of 4 BALB/c mice. The cells were suspended in RPMI 1640 culture media without serum supplement. The mice were immunized once a week for 3 weeks and on four consecutive days during the fourth week. The mice were sacrificed 24 hours after the last injection on the 4th week and their spleens were removed.

The spleens were gently teased apart in Dulbecco's minimum essential medium (DMEM), a tissue culture media which may be obtained from Gibco, Grand Island, New York, with no serum supplement. The single cell suspension was washed, concentrated, counted and mixed in a 2:1 ratio with myeloma cells (P3xAg8.653) derived from the BALB/c mouse and obtained from the ATCC. This particular myeloma line does not secrete any antibody.

The cell mixture was centrifuged in a 10 ml round bottom tube, the media was aspirated and 0.2 ml of 30% PEG 4000 (Sigma) in Hanks balanced salt solution (HBSS) which may be obtained from Gibco, Grand Island, New York, containing 3mM EDTA was added to the pellet for 1 minute. This solution was rapidly diluted with 10 ml of serum free-DMEM and centrifuged at 1200 RPM for 5 minutes. The cells were resuspended in DMEM supplemented with 20% fetal bovine serum (FBS) and were incubated in a 75 cm$^2$ flask overnight. The following day, 17 x 24 well plates were seeded with 2 x 10$^5$ cells per well in HAT supplemented DMEM plus 20% FBS. The results from this fusion are shown in Table 1.

### EXAMPLE 2b

Hybridoma Generation (Membrane Extract Antigen):

From the tumor cells obtained in Example 1b above, tumor cell membranes were prepared with a motorized Dounce homogenizer which may be obtained from Fisher Scientific in 0.01m Dulbecco's phosphate buffered saline with 10 units/ml of DNAase I and 0.1% aprotinin (PBS-H) (Sigma Chemical Co., St. Louis, MO). The extract was then incubated at 37°C for 30 minutes, layered onto 41% sucrose/PBS-H and centrifuged at 100,000 xg for 60 minutes; crude membranes were isolated at the interface. Membranes were then washed twice with PBS with 0.1% phenylmethyl sulfonyl fluoride and twice with PBS. Mice were immunized subcutaneously with one injection of 10 mg (total protein) of the membranes for six weeks. Spleen cells were isolated at the end of the six week immunization. The fusion was carried out at a 10:1 ratio of spleen cells to NS-1 mouse myeloma cells (BALB/c derived) obtained from the ATCC using 50% PEG 1000. Hybrids were selected in hypoxanthine, aminopterin, and thymidine (HAT) supplemented medium using both 24-well and 96-well plating technique. Selected hybridomas were cloned by either a limiting dilution or a serial dilution technique on feeder layers of freshly isolated thymocytes from three-to-four-week-old mice, or in wells supplemented with spent culture medium from NS-1 cells. Fusion resulted in 70-90% growth in the wells plated. The results of this fusion are illustrated in Table 1.

## TABLE 1

| Immunogen | Hybrids[1] | Tumor Reactive[2] | Nonreactive to control |
|---|---|---|---|
| Whole Cells[3] | 104 | 101 | 76[5] |
| Membranes[4] | | | |
| FMH59 | 238 | 31 | 10[6] |
| FMH42 | 317 | 48 | 17[6] |

1. Numbers of wells positive for hybrid growth.

2. Numbers of subcloned positive wells.

3. Biotherapeutics, 1985.

4. Morgan et al, Hybridoma 3, 233-245 (1989).

5. Human fibroblasts.

6. T and B cells.

## EXAMPLE 2c

Hybridoma Generation (Immunization of Animals with Interferon Treated Human Tumor Cells):

Four week old BALB/c mice were immunized with $10^7$ human tumor cells prestimulated with gamma interferon as described in Example 1c(2). Tumor cells were injected intraper-itoneally. Mice were immunized with this same number of tumor cells at day 30 and day 60.

## EXAMPLE 2d

Hybridoma Generation (Immunization of Animals):

(1) Rescued Idiotype Immunization:

On day one, 25-50 micrograms of rescued idiotype in a 1:1 or 1:2 dilution of PBS:CFA (phosphate buffered saline: complete Freund's adjuvant) was injected intraperitoneally into BALB/c mice. A total volume of no more than 0.2ml per animal was given.

On day 14, 25-50 micrograms of rescued idiotype was given intravenously (tail vein) in 0.2ml to 0.3ml PBS.

On day 17 or 18 (72-96 hours later) animals were bled for control serum or were sacrificed and their spleens harvested for a fusion.

It was found that hyperimmunization beyond day 14 could be accomplished by substituting 25-50 micrograms of rescued idiotype in a 1:2 dilution of PBS:IFA (phosphate buffered saline: incomplete Freund's adjuvant) and injecting no more than 0.2ml/animal. Although, this can be used as a boost, it was found to be less satisfactory and 14-28 days should elapse before continuing with the intravenous boost presented above.

(2) Alternative Rescued Idiotype Immunization:

The immunogen was prepared at a concentration of 1mg purified idiotype per ml in 150mM saline. The immunogen was then frozen in aliquots for subsequent immunization. An emulsion of immunogen 1:1(v:v) in CFA which may be obtained from Difco, Detroit, Michigan, was injected at 450 microliters/mouse (225 micrograms protein) as follows:   200 microliters intraperitoneally, 50 microliters subcutaneously in each footpad, and 50 microliters subcutaneously at the base of the tail.  After 30 days mice were reimmunized as above with an emulsion of immunogen 1:1 in IFA which may be obtained from Difco, Detroit, Michigan.  Mice were then sacrificed after 4 days for fusion or were hyperimmunized over a longer period of time.

(3) Whole Cell Immunization:

On day 1, 7, 14 and 28, 2 x $10^6$ viable cells in 0.5 ml RPMI 1640 were injected intraperitoneally into BALB/c mice. Animals were then rested from 14 to 28 days.  On the chosen day, 1 x $10^6$ cells in 0.2ml to 0.3ml PBS were injected intraveneously (tail vein) into immunized mice.  Animals were then bled for control serum 88 to 96 hours later or were sacrificed and their spleens harvested for fusion.

(4) Tumor Lysate Immunization (with human immunoglobulin as target):

A tumor sample was completely lysed and solubilized in 50mM n-octyl$\beta$-D-gluco-pyranoside (N-OGP) detergent which may be obtained from Sigma Chemical Co., St. Louis, Missouri. This solution was centrifuged at 10,000 x g for 20-30 minutes and the supernatant was passed over a goat anti-human immunoglobulin affinity column.  Rescued immunoglobulin was eluted with glycine sulfate pH 2.3 in 50mM N-OGP.  Eluate was collected in 5ml aliquots with 1ml of 1M Tris/50mM N-OGP. Absorbances at 280 nm were read and tubes bearing immunoglobulin were pooled and dialyzed against extensive changes of PBS at 4°C for several days.  Human immunoglobulin rescued in this fashion was then treated like rescued idiotype and protocol (1) above was followed.

### EXAMPLE 2e

Hybridoma Generation (Anti-idiotype Fusions):

Monoclonal antibodies against idiotype were produced by fusing immunized mouse lymphocytes with the murine myeloma Sp2/0-Ag14 which was obtained from the ATCC.  BALB/c mice were immunized with whole cells containing idiotype, with idiotype eluted directly from lysed whole cells over a goat anti-human (GAH) Ig affinity column (GAH Ig may be obtained from Cappel Worthington, Malvern, PA), or with idiotype purified from cell supernatant or ascites following a rescue fusion.

Fusion parent cells (Sp2/0 Ag14) were grown and collected, washed 3 times in RPMI-1640, counted, and stored on ice. Spleen (and axial lymph nodes depending on immunization protocol), were removed, a single cell suspension was prepared, washed 3 times in RPMI-1640, counted, and stored on ice. Appropriate dilutions were prepared at $3 \times 10^7$ splenocytes and $1 \times 10^7$ Sp2/0 cells per 15ml round bottom tube.

After centrifuging for 5 minutes at 250 x g all media was carefully removed. 0.2ml of 35% PEG (polyethylene glycol 1450, Aldrich) was added per tube for 1 minute at room temperature and sufficient agitation was used to resuspend the pellet in PEG. The PEG was quickly diluted with 10ml RPMI-1640 and was let stand for 10 minutes at room temperature. Fusion tubes were centrifuged for 5 minutes at 250 x g and all dilute PEG was carefully removed. Each pellet was gently resuspended in 5ml RPMI-1640 with 15% fetal bovine serum, the total volume was brought to 21 or 31ml in a 50ml conical, and was plated at 0.1ml/well in two or three 96 well tissue culture plates. The plates were incubated overnight at 37°C in a humidified chamber with 6% $CO_2$. After 18 to 24 hours 0.1ml of 2 x HAT (hypoxanthine, aminopterin, thymidine) was added per well. Single colony wells were screened for secretion of antiidiotypic antibodies after 2 to 3 weeks. Forty percent of the wells were found to secrete antiidiotypic antibodies.

### EXAMPLE 3a

Monoclonal Antibody "Cocktail" Preparation (Whole Cell ELISA Screening System):

Hybridomas produced in Examples 2a and 2b above were selected for production of antibody with tumor selectivity and non-cross reactivity with normal tissues and patients' peripheral blood cells.

Normal tumor and patient blood cells were prepared in a suspension and attached to 96 well plates pretreated with poly-L-lysine (5 mg/100 ml in PBS). The cells were fixed by treating them with 0.1% glutaraldehyde for three minutes. After washing and blocking with 100mM Glycine plus 0.1% BSA in PBS, the plates were washed and non-specific protein binding was blocked by the use of 1% BSA-PBS. At this point plates were ready to use or could be frozen in the presence of 1% BSA. Controls include: culture media blank (as tested), a negative control usually non-specific mouse IgG and a positive control using monoclonal mouse IgG anti-human fibronectin. This last control gives a positive against all attachment cells tested and not only provides a tissue control but also a system (reagent) control.

Controls and samples (undiluted culture supernatants) were incubated at room temperature for 45 minutes, washed and the plates were then incubated for 1 hour with goat anti-mouse IgG (H+L chains) conjugated to peroxidase (diluted 1:5000). After washing, OPD color reagent (Ventrex, Portland, MA) was added and color developed over 15-30 minutes. The reaction was stopped with 2.5M NaOH, and the plates were read on a dual beam ELISA (Dynatech or Bio Tek) reader.

Selected antibodies were checked to see if they react with different epitopes of the tumor-associated antigen (TAA). At this point two choices were available to produce an antibody cocktail: (1) Produce monoclonal antibodies by each selected clone using ascites production techniques ($1-3 \times 10^6$ cells/animal) or tissue culture methods (Ventrex or Monsanto/Queue Systems Mouse), and mix the individually produced antibodies at desired ratios to produce the cocktail, or (2) Mix the hybridomas in equal numbers and grow them either in ascites or using tissue culture methods to produce the cocktail at one time. The latter method produces a cocktail where the ratio for the antibodies is not predetermined, while the former method gives a desired mixture of each antibody in the cocktail.

## EXAMPLE 3b

Monoclonal Antibody "Cocktail" Preparation (Whole Cell Filter Screen):

A vacuum was slowly applied to a 96-well plate with filters seated in the wells (filter plates are provided by V & P Scientific, Inc.). 50 microliters of FBS buffer (10% FBS, 1% BSA, 0.3% swine skin gelatin, 0.01% thimerosal in 0.01M PBS) was added to each well for at least 10 minutes to block the filters. The buffer was removed by suction to reseat filters. 150,000 to 200,000 viable target cells diluted in FBS buffer were added to each well (50-100 microliters/well) and was seated on the filters by suction. Vacuum was continued and wells were washed three times in gelatin buffer (0.3% gelatin in 0.01M PBS). To block non-specific binding sites, 50 microliters of a 50% normal goat serum/50% FBS buffer was added to each well and was incubated at 37°C for 60 minutes. The plate was washed three times with gelatin buffer, sealed with parafilm and 100 microliters of culture supernatant was added to the appropriate well(s). The plate was again incubated at 37°C for 60 minutes after which time it was washed and sealed as above. Alkaline phosphatase conjugated goat anti-mouse Ig antibody was diluted 1:500 in a BSA buffer (1% BSA, 0.3% gelatin, 0.01M PBS) and was added to each well at 100 microliters per well. This was incubated at 37°C for 60 minutes then washed five times with gelatin buffer. Two hundred microliters of diethanolamine substrate was added to each well and then the plates were sealed. Reaction was stopped after 30-60 minutes with 1N NaOH and 100 microliters of each well was transferred to a vinyl assay plate from which absorbances were read.

## EXAMPLE 3c

Monoclonal Antibody "Cocktail" Preparation (Cell Lysate Screen):

The cell lysate screen is used to screen hybridomas for secretion of anti-idiotypic monoclonal antibodies.

According to this process, plates were coated overnight at 4°C with 50 microliters of goat anti-human Ig

-28-

0222360

polyvalent at 10 $\mu$ g/ml PBS. The plates were then washed three times with 2% BSA/PBS with each wash remaining on the plate for 3 minutes. Target cells were lysed and solubilized in 2% BSA/0.5% NP-40/PBS for 30 minutes at 4°C at a concentration of 5 x $10^6$ cells/ml. This lysate was centrifuged at 2000 RPM for 10 minutes after which 50 microliters was added to each well. This mixture was allowed to incubate at 37°C for one hour. The plates were then washed 3 times for 3 minutes each with 1% BSA/0.05% Tween 20/PBS. Culture supernatants were added at 50 microliters per well and were allowed to incubate for one hour at 37°C. The plates were washed 3 times for 3 minutes each with 1% BSA/0.05% Tween 20/PBS. Fifty microliters of 1:500 dilution of goat F(ab')$_2$ anti-human IgG (gamma specific) diluted in 0.1% BSA/0.05% Tween 20/PBS was added to each well and was allowed to incubate at 37°C for one hour. The plates were washed 3 times as above and 3 times with tap water, but without 3 minute waits between the tap water washes. 200 microliters of standard diethanolamine substrate was added to each well and was allowed to incubate for at least one hour. Reaction was stopped with 1N NaOH (50 microliters/well), 100 microliters/well was transferred to a flexible vinyl assay plate and absorbances were read.

### EXAMPLE 3d

Monoclonal Antibody "Cocktail" Preparation (Screening of Antibodies for Interferon Augmentable Activity Against Human Tumor Cells):

Human tumor cells were incubated in gamma interferon under conditions identical to those used prior to animal immunization as in Example 1c(2). Tissue culture supernatants were harvested from hybridoma clones secreting potential antitumor antibody. Human tumor cells were immobilized on filters in microtiter wells and supernatants were tested against untreated and interferon stimulated tumor cells. Antibodies demonstrating augmented binding to tumor cells treated with interferon were selected for subcloning and additional testing.

The monoclonal antibodies produced and selected for positive activity in any of Examples 3(a)-(d) can be combined to produce a monoclonal antibody cocktail having a number of different types of antibodies therein.

## EXAMPLE 4a

Immunoconjugate Generation (Linkage with Toxin):

Abrin, obtained from Calbiochem (La Jolla, CA), Sigma Chemical Co. (St. Louis, MO) or through courtesy of O. Fodstad (Oslo, Norway) was reduced with 5% mercaptoethanol. The A and B chain subunits were separated by two cycles of affinity chromatography, using galactose-substituted agarose followed by lactose-substituted agarose. The abrin A-chain passed through the first column while the B chain and intact toxin were retained when PBS plus 0.05% mercaptoethanol was used as the solvent. The isolated A chain was shown to be free of B chain and intact toxin by SDS-PAGE. The monoclonal antibody was mixed with N-succinimidyl-3-(2-pyridyldithio)propionate (SPDP) (Pharmacia, Boston, MA) at room temperature for 30 minutes followed by dialysis with PBS in cold for 16 hours. Under the conditions used, 2 to 3 moles of 2-pyridyldithiopropionate (PDP) were attached per mole of antibody, as determined by an increase in absorbance at 343 nm upon reduction by dithiothreitol (DTT). The PDP-substituted monoclonal antibody was then allowed to react for 6 hours at room temperature with the abrin A chain that was reduced with 0.1M DTT and isolated on Sephadex™ G-25 (Pharmacia) just prior to use. The immunoconjugates were isolated by gel filtration using Sephadex™ G-150 (Pharmacia) and were identified on SDS-PAGE (8% polyacrylamide gel).

## EXAMPLE 4b

Immunoconjugate Generation (Linkage with Toxin):

The antibody 81A6 with selective reactivity for neuroendocrine tissue was conjugated with the purified A chain of ricin. Purified ricin alpha-chain was obtained from E Y Laboratories, Inc., San Mateo, CA. Contaminating whole ricin and B-chain were further removed by passage of the ricin preparation over an immuno-affinity column with mouse monoclonal antibody to the B-chain of ricin coupled to Sepharose™. Dithiothreitol (DTT) was added to the ricin A-chain solution to a final concentration of 50 millimolar and incubated for one hour at room temperature. Ricin A-chain preparation was then dialyzed against PBS overnight at 4°C with three changes of buffer, and concentrated to 4mg per ml with an Amicon concentrator (Amicon Corp., Danvers, MA). The 81A6 antibody was dialyzed into 0.1 molar of sodium chloride, 0.1 molar sodium phosphate buffer, pH 7.4. N-succinimidyl-3-(2-pyri-dyldithio) propionate (SPDP) was prepared in solution of absolute ethanol and added to the dialyzed 81A6 in a 10 fold molar excess. The mixture was stirred for 30 minutes at room temperature and dialyzed against phosphate buffered saline, pH 7.4. Freshly reduced ricin A-chain solution was mixed with the SPDP antibody preparation in a 5 fold molar excess (equal weights of ricin A-chain and antibody). After a 4°C overnight incubation, immuno-conjugate was separated from unconjugated A-chain by passage over Sephacryl™ S200 (Pharmacia). This immunoconjugate was then demonstrated to selectively inhibit protein synthesis by small cell cancer cells.

### EXAMPLE 4c

Immunoconjugate Generation (Linkage with a Biological):

The monoclonal antibody 81A6 reactive with neuroen-docrine tissue can be conjugated with gamma interferon by the method described by Alkan et al, J. Interferon Research, Vol. 4, No. 3, 355-363, 1984. According to this method, the 81A6 antibody would be placed in PBS at 6mg per ml. Affinity purified gamma interferon would be placed at $2 \times 10^6$ units per ml to both antibody and interferon solutions. Eight microliters of 20 millimolar SPDP would be added for 20 minutes at room temperature. The SPDP antibody solution would then be dialyzed against PBS and then against sodium acetate buffer pH 4.5 and then incubated with 25 millimolar dithiothreitol (DTT) for 15 minutes at room temperature. The interferon-SPDP solution would also be dialyzed against PBS. The interferon derivative and the thiolated 81A6 derivative would be mixed and incubated for two hours at room temperature and overnight at 4°C. The mixture would then be passed over Sephacryl S200. The fraction containing antibody and interferon activity would be separated from unconjugated interferon.

## EXAMPLE 4d

Immunoconjugate Generation (Linkage with a Radioactive Isotope):

One mg of a T101 antibody preparation was labeled with diethylenetriaminepentaacetic acid (DTPA) to give about 2 molecules of DTPA per antibody molecule. The antibody-DTPA conjugate was supplied by Hybridtech, La Jolla, CA. The antibody-DTPA conjugate was mixed with 5 mCi $^{111}$In. Excess DTPA was added to remove free $^{111}$In from the final conjugate. Doses of 1 mg were administered to patients for radioimaging. Selected patients were injected with 9 or 49 mg of unlabeled "cold" T101 simultaneously with the radiolabeled T101. The immunoconjugate preparation was found to localize in the patient's skin and lymph nodes. A control monoclonal antibody (9.2.27) (National Cancer Institute, Fredrick, MD), $^{111}$In labeled, that reacts against human melanoma cells did not localize in the lymphoma demonstrating the selectivity of this approach.

## EXAMPLE 4e

Immunoconjugate Generation (Linkage with a Radioactive Isotope):

The method of covalently attaching a strong chelator, diethylenetriaminepentaacetic acid (DTPA), Krejcarek et al, Biochem Biophys. Res. Comm., 77:581 (1977) and Wagner et al, J. Nucl. Med., 20:428 (1979), to immunoglobulin G (IgG) antibody and subsequent labeling of the coupled protein with $^{111}$In was used. All reagents were ultrapure and glasswares, etc. were metal free.

First, DTPA was coupled to monoclonal antibody 81A6. A modified method of Hnatowich et al, Int. J. Applied Radiation Isotopes, 33:327 (1982), from a personal communication of

-32-

0222360

Beverly A. Brown (Du Pont, Immunopharmaceutical Research, No. Billerica, MA) was used. 81A6 (5-10ml, 6mg/ml) was dialyzed with Spectrapure membrane tubing (Spectrum Medical Ind. Inc., Los Angeles, CA) N'-2-Ethane of HEPES (N-2-Hydroxyethylpiperazine-Sulfonic Acid, Gibco, Grand Island, NY) buffer (25mM HEPES + 250mM NaCl, pH adjusted to 7.0 with 6N NaOH) for 4 hours or overnight at 4°C. The volume and U.V. absorption at 280 nm were measured to determine the amount of coupled 81A6. The amount of 10-40 times molar excess of DTPA anhydride (Sigma Chemical Co., St. Louis, MO) was added to proceed at room temperature with agitation for 30 minutes. The coupled antibody was purified by dialysis against HEPES buffer at 4°C for 2 days. Volume and U.V. absorption at 280 nm were measured to determine the amount of coupled 81A6. It was immediately stored at 4°C before labeling.

Next, the DTPA-81A6 couple was labeled with [111]In. Labeling was accomplished by adding equal volume of 0.5 M acetate solution of $^{111}$In to the coupled antibody solution. The volume of $^{111}$In which contains the desired amount of radioactivity was determined. For every 30 microliters of $^{111}$In used, 20 microliters of 1.0 N HCl were added to prevent colloid formation. Then 50 microliters of 1.0 N sodium acetate were added to make 0.5 M acetate solution. This fresh [111]InAc was mixed with 100 microliters of DTPA-81A6 at room temperature for 30 minutes. Then $^{111}$In labeled DTPA-81A6 was purified on a Sephadex G25M column (Pharmacia, Uppsala, Sweden) with phosphate buffered saline (Gibco Laboratories, Chagrin Falls, OH) eluate. The radioactivities of eluted fractions showed only one peak. The peak fractions were collected to inject to nude mice with small cell tumors. From our data 0.2ml of injectate consisting of a range from 7 mCi to 20 mCi of $^{111}$In and 50 micrograms to 140 micrograms of antibody gave good imaging results.

While the present invention has been described in detail and with reference to specific examples thereof, it will be apparent to one skilled in the art that various changes and modifications can be made thereto without departing from the spirit and scope thereof as defined by the following claims.

What is claimed is:

1. A method of producing a patient-specific cytotoxic reagent for the treatment of a malignant tumor, comprising:

(a) processing and expanding cells from the tumor to provide a tumor cell preparation,

(b) immunizing B-lymphocytes against said tumor cell preparation to produce antibodies selective for the tumor cells,

(c) fusing said immune B-lymphocytes with fusion partner cells to form a plurality hybridomas,

(d) testing each of said plurality of hybridomas for production of an antibody selective for said tumor cells,

(e) cloning a plurality of said hybridomas to isolate those which generate said selective antibodies,

(f) combining said monoclonal antibodies to produce a monoclonal antibody cocktail that will react with substantially all of said tumor cells, and

(g) linking each said monoclonal antibody in said monoclonal antibody cocktail with a substance which is toxic to the tumor cells to generate an immunoconjugate reagent for treating the tumor.

2. The method of Claim 1 wherein the tumor cells are processed by culturing said cells in vitro.

3. The method of Claim 1 wherein the tumor cells are processed by growing said cells in vivo.

4. The method of Claim 1 wherein the tumor cells are processed by making an antigen preparation of the membranes of the tumor cells.

5. The method of Claim 1 wherein the tumor cell preparation is injected into an animal to immunize the animal and generate said B-lymphocytes.

6. The method of Claim 5 wherein said B-lymphocytes are removed from the animal and fused with said fusion partner

-35-

cells.

0222360

7. The method of Claim 1 wherein the tumor cell preparation is cultured in vitro with reactive cells to produce immunized B-lymphocytes.

8. The method of Claim 1 wherein said B-lymphocytes are generated spontaneously by the patient having the malignant tumor.

9. The method of Claim 1 wherein said fusion partner cells are myeloma cells.

10. The method of Claim 1 wherein said fusion partner cells are B-lymphoblastoid cells.

11. The method of Claim 1 wherein said fusion step is accomplished by electrofusion.

12. The method of Claim 1 wherein said step of testing said hybridomas for production of monoclonal antibodies against said tumor cells comprises culturing antibody containing said hybridomas in vitro and testing for binding with the supernatants from tumor cells.

13. The method of Claim 1 wherein said cloning step comprises selecting and isolating a plurality of hybridomas which each produce an antibody reactive to the tumor cells, and producing a hybridoma preparation of said plurality of hybridomas.

14. The method of Claim 13 wherein said hybridoma preparation is produced by combining from 3 to 10 unique types of hybridomas which each produce an antibody specific to the tumor cells thereby creating a hybridoma preparation which will produce a monoclonal antibody cocktail that will bind with substantially all the tumor cells.

15. The method of Claim 1 wherein said substance combined with said selective monoclonal antibodies is a drug.

16. The method of Claim 1 wherein said substance combined with said selective monoclonal antibodies is a toxin.

17. The method of Claim 1 wherein said substance combined with said selective monoclonal antibodies is a radioactive isotope.

18. The method of Claim 1 wherein said substance combined with said selective monoclonal antibodies is a biologic substance.

19. The method of Claim 1 further comprising the step of producing a quantity of said monoclonal antibody cocktail large enough for therapeutic use on the tumor cells.

20. The method of Claim 18 further comprising the step of purifying said quantity of monoclonal antibody cocktail.

21. A method of producing a serum for the treatment of a malignant tumor of a patient, comprising:
(a) processing the cells from the tumor to produce a preparation for injection,
(b) administering said preparation to an animal to immunize B-cells in the animal,
(c) removing said immunized B-cells from the animal,
(d) fusing said immunized B-cells with myeloma cells to form a plurality of types of hybridomas,
(e) testing each of said plurality of hybridomas for the production of an antibody,
(f) exposing each said antibody to the tumor cells to determine a plurality of selective antibodies which are reactive to the tumor cells,
(g) cloning said hybridomas which produce said plurality of selective antibodies,
(h) combining said plurality of selective antibodies to produce a monoclonal antibody cocktail that will react with substantially all of the tumor cells, and

0222360

(i) linking each said plurality of selective antibodies in said monoclonal antibody cocktail with a substance which is toxic to said tumor to form an immunoconjugate preparation for treating the tumor.

22. The method of Claim 21 wherein said step of processing the tumor cells comprises culturing human tumor cells in vitro in animal tissue to promote growth of said cells.

23. The method of Claim 21 wherein said step of processing the tumor cells comprises implanting human tumor cells in vivo in an animal host, growing said implanted tumor cells in the animal and then removing said grown tumor cells or fluid associated therewith from the animal.

24. The method of Claim 21 wherein said step of administering said preparation comprises injecting said preparation into the animal.

25. The method of Claim 21 wherein said step of administering said preparation comprises injecting treated cancer cells from the patient into the body of the patient.

26. The method of Claim 21 wherein said step of removing the immunized B-cells comprises extracting said B-cells from an organ in the animal.

27. The method of Claim 21 wherein said fusing step is accomplished by electrofusing each said B-cell to one of said myeloma cells.

28. The method of Claim 21 wherein said step of exposing each said antibody to the tumor cells comprises identifying each type of hybridoma which produces an antibody selective for at least one antigen on one of said tumor cells.

29. The method of Claim 21 wherein said step of exposing said antibody to the tumor cells comprises generating at least three antibodies which are each selective for a different antigen on the tumor cells.

30. The method of Claim 29 wherein said cloning step comprises cloning each of said hybridomas which produces one of said at least three antibodies.

31. The method of Claim 21 wherein said combining step comprises chemically bonding said antibodies with a drug which is reactive to said tumor cells.

32. The method of Claim 21 wherein said combining step comprises chemically bonding said antibodies with a toxin which is reactive to said tumor cells.

33. The method of Claim 21 wherein said linking step comprises chemically bonding said antibodies with a radioactive isotope which is reactive to the tumor cells.

34. The method of Claim 21 wherein said linking step comprises chemically bonding said antibodies with a biologic substance which is reactive to the tumor cells.

35. A method of biologically treating a cancerous tumor comprising:
(a) generating a preparation from cells of said tumor;
(b) applying said preparation to B-lymphocyte cells to generate immunized B-lymphocyte cells;
(c) fusing each said immune B-lymphocyte cells with a fusion partner to form hybridomas;
(d) selecting said hybridomas which produce antibodies selective for the tumor cells;
(e) combining the antibodies of said selected hybridomas with a substance which is reactive to said tumor cells to generate an immunoconjugate; and
(f) administering said immunoconjugate in therapeutic doses to treat the tumor.

36. A method of biologically treating a cancerous tumor comprising:

(a) processing cells from the tumor to generate a preparation for injection;

(b) immunizing B-cells of an animal by injecting said preparation;

(c) removing said immunized B-cells from the animal;

(d) fusing said immunized B-cells with animal myeloma cells to form hybridomas;

(e) testing each of said hybridomas to select hybridomas which produce an antibody selective for the tumor cells,

(f) cloning a plurality of said selected hybridomas producing antibodies which are selective for said tumor cells;

(g) combining said specific antibodies to produce a monoclonal antibody cocktail that will react with substantially all of the tumor cells;

(h) linking each said specific antibodies of said monoclonal antibody cocktail with a substance which is toxic to the tumor to form an immunoconjugate solution; and

(i) testing said immunoconjugate solution for localization and effectiveness against the tumor cells; and

(j) applying said immunoconjugate solution in therapeutic doses against the tumor.

37. A patient-specific monoclonal antibody preparation including a combination of antibodies each being selective for at least one of the cell types of a tumor, the combination being collectively selective for substantially all of the cell types of said tumor.

38. The antibody preparation of Claim 37 wherein each antibody is produced from a cell immunized against said tumor.

39. The antibody preparation of Claim 37 wherein said combination of antibodies are produced by a plurality of hybridomas each being derived from a B-lymphocyte immunized against said tumor.

40. The antibody preparation of Claim 39 wherein each said plurality of hybridomas is formed by fusion of a B-lymphocyte with a myeloma cell.

41.   The antibody preparation of Claim 37 wherein said antibodies are combined with a biological substance which is toxic to substantially all of the cell types of the tumor to form an immunoconjugate preparation.

42.   The antibody preparation of Claim 41 wherein said immunoconjugate preparation is formed by chemically linking said biological substance to the antibodies.

43.   The antibody preparation of Claim 41 wherein said biological substance is selected from the group consisting of a drug, toxin or radioactive isotope.

FIGURE 1